# EUROPÄISCHE PATENTSCHRIFT

(11) **EP 0 861 128 B1**
(45) Veröffentlichungstag und Bekanntmachung des Hinweises auf die Patenterteilung: **19.02.2003**
(21) Anmeldenummer: 96934570.1
(22) Anmeldetag: 08.10.1996
(51) Int. Cl.: B05B 11/00, B05B 11/04

(54) **VOR KONTAMINATION SCHÜTZENDE ABGABEVORRICHTUNG FÜR FLUIDE**
FLUIDS DISPENSER DESIGNED TO PROTECT THE CONTENTS FROM CONTAMINATION
DISTRIBUTEUR DE FLUIDES PROTEGEANT CES DERNIERS CONTRE UNE CONTAMINATION

(30) Priorität: 17.11.1995 DE 19542959; 13.03.1996 DE 19609880
(43) Veröffentlichungstag der Anmeldung: 02.09.1998
(73) Patentinhaber: URSATEC VERPACKUNG GMBH, 66129 Saarbrücken (DE)
(72) Erfinder: GEIMER, Günter, D-66901 Schönenberg-Kübelberg 2 (DE)
(74) Vertreter: Körber, Wolfhart, Dr. rer.nat.
(86) Internationale Anmeldenummer: EP9604356
(87) Internationale Veröffentlichungsnummer: WO97018902

(56) Entgegenhaltungen:
- WO-A-94/11115
- US-A- 5 154 325
- US-A- 5 232 687

## Beschreibung

Die Erfindung betrifft eine Abgabevorrichtung für Fluide aus einem Vorratsbehälter, bei der das Fluid durch einen Fluidausgang mit Hilfe einer einen Kolben aufweisenden Pumpe abgegeben wird und bei der während der Abgabe des Fluids Luft zum Druckausgleich in den Vorratsbehälter durch einen Lufteingang einströmt, der die Pumpe (2) umgibt und in dem ein Sterilfilter zur Sterilisation, Entkeimung oder Keimreduktion der Luft vorgesehen ist, wobei der Lufteingang durch einen eng ausgebildeten Spalt (19) zwischen dem Umfang des Kolbens (3) der Pumpe (3) und einem Gehäuseabschnitt (18) gebildet ist.

Eine solche Abgabevorrichtung ist beispielsweise aus der US-A-4,694,976 bekanntgeworden.

Bekannt sind herkömmliche Abgabevorrichtungen bzw. Dosierpumpen für Pharmazeutika oder Kosmetika. Derartige Abgabevorrichtungen sind auf einem Vorratsbehälter für das abzugebende Fluid befestigt. Um das Entstehen eines Unterdrucks bei der Abgabe des Fluids in dem Vorratsbehälter zu vermeiden, strömt Umgebungsluft in den Vorratsbehälter ein. Der Nachteil solcher Abgabevorrichtungen ist, daß die einströmende Luft verkeimt ist und somit das Fluid im Vorratsbehälter kontaminiert. Des weiteren verkeimt das Fluid an der Austrittsöffnung durch Kontakt mit der Umgebung. Diese Verkeimung kann durch den Austrittsweg des Fluids in den Vorratsbehälter zum Vorratsfluid durchwachsen und dieses kontaminieren. Eine solche Kontamination führt bei Pharmazeutika und Kosmetika zum Verderb und zur Gefahr für den Anwender.

Aus diesem Grund sind sogen. "Airless-Systeme" entwickelt worden, bei denen ein Druckausgleich im Vorratsbehälter nicht erforderlich ist, d.h. keine Umgebungsluft in den Vorratsbehälter einströmen muß. Dieses wird durch einen speziellen Vorratsbehälter erreicht. Es gibt Vorratsbehälter mit Schleppkolben, bei denen das Volumen des Vorratsbehälters bei der Fluidabgabe durch einen Schleppkolben verringert wird. Nachteilig ist hierbei, daß nur zylinderförmige Vorratsbehälter verwendet werden können. Des weiteren gibt es doppelwandige Vorratsflaschen, bei denen sich das Vorratsfluid in einem verformbaren Innenbeutel befindet und einströmende Umgebungsluft für den Druckausgleich zwischen einem festen Außenbehälter und einem verformbaren Innenbehälter aufgenommen wird. Ferner gibt es Vorratsbehälter, in denen keimfreie Luft unter erhöhtem Innendruck aufgenommen ist. Nachteilig an all diesen Systemen ist, daß man für die Abgabevorrichtung ein spezielles Behältnis benötigt. Dies macht das System sehr aufwendig in der Herstellung und nicht anpassungsfähig an bereits vorhandene herkömmliche Vorratsbehälter und Abfüllsysteme. Weiterhin ist ein solches System sehr teuer, so daß es damit nicht möglich ist, Pharmazeutika und Kosmetika im unteren Preisniveau ohne Konservierungsmittel anzubieten. Konservierungsmittel haben unabhängig vom Preisniveau der Produkte für den Verbraucher schädliche Wirkungen. Sie sind bedenkliche Substanzen und führen zu unerwünschten Nebenwirkungen.

Die Erfindung geht von der Erkenntnis aus, daß bei Abgabevorrichtungen der eingangs genannten Art, bei denen der Fluidausgangsweg und der Lufteingangsweg voneinander getrennt geführt sind, für die Entkeimung des Fluids bzw. der Luft unterschiedliche Methoden verwendet werden können. Der Erfindung liegt die Aufgabe zugrunde, eine Vorrichtung der eingangs genannten Art zur Mehrfachabgabe von Fluiden zu schaffen, bei der das Vorratsfluid vor Kontamination mit Keimen ohne Verwendung eines Konservierungsmittels geschützt werden kann und für den Druckausgleich durch Luft sichergestellt ist, daß keine Druckausgleichs-Luft in den Behälter gelangen kann, die nicht durch den Sterilfilter geströmt ist.

Diese Aufgabe wird durch die Merkmale der unabhängigen Ansprüche 1 oder 2 gelöst. Zweckmäßige weitere Ausgestaltungen ergeben sich aus dem Unteransprüchen.

In der gattungsbildenden US-A-4,694,976 ist eine Keimfreihaltung des Fluids insbesondere durch Substanzen mit oligodynamischer Wirkung nicht beschrieben. Der Lufteintritt für den Druckausgleich umgibt mit einer Kammer zwar die Pumpeinrichtung, der Sterilfilter ist jedoch seitlich im Gehäuse angeordnet. Damit ist nicht auszuschließen, daß ungefilterte Luft in den die Pumpeinrichtung umgebenden Raum angesaugt wird wenn der Pumpenkolben bzw. dessen Stößel gegenüber dem Gehäuse bewegt wird. Demgegenüber sieht die Erfindung vor, daß der Sterilfilter gleichzeitig als Abdichtung der bewegten Pumpenteile gegenüber dem Gehäuse dient.

Die vorliegende Erfindung verwendet eine Abgabevorrichtung für Fluide, bei der der Fluidausgangsweg zumindest in einem Teil getrennt von dem Lufteingangsweg für die Luft, die zum Druckausgleich in das Vorratsgefäß gelangen soll, geführt ist. Auf diese Art und Weise können die Luftkeime und Fluidkeime getrennt voneinander unwirksam gemacht werden. Dies hat den Vorteil, daß für die Entkeimung des Fluids bzw. der Luft unterschiedliche Methoden verwendet werden können, die jeweils an die verschiedenen Bedürfnisse angepaßt werden können. Die Entkeimung oder Keimreduktion des Fluids geschieht im Bereich des Austrittsweges desselben durch oligodynamisch wirksame Substanzen. Es können Schwermetall und/oder Schwermetall-Legierungen in metallischer und/oder ionischer Form verwendet werden.

Als besonders vorteilhaft hat sich die Verwendung von Silber gezeigt. Die Entkeimung oder Keimreduktion des Fluids im Bereich des Austrittsweges des Fluids bewirkt, daß keine Keime von der mit der Umgebung in Berührung kommenden Austrittsöffnung entgegen der Fließrichtung des Fluids am Ventil vorbei (z.B. durch Wachstum) in den Vorratsbehälter gelangen können und daß das keimfreie Fluid aus dem Vorratsbehälter bei der Ausbringung nicht mit kontaminierten Restmengen (z.B. ander Austrittsöffnung) kontaminiert wird und die mikrobiologische Qualität dadurch für den Verbraucher bedenklich wird. Weiterhin wird ein Wachstum der Keime an der Austrittsöffnung und in allen Bereichen der Abgabevorrichtung verhindert, insbesondere bei einer Pumpe oder einem Ventil.

Die Entkeimung der zum Druckausgleich in den Vorratsbehälter einströmenden Luft kann desweiteren durch andere auf die Luftkeime abgestimmte Methoden erlangt werden. So kann die Luft auf ihrem Eintrittsweg durch einen Sterilfilter geführt werden oder durch Permeation durch eine im Luftweg befindliche Membran in den Vorratsbehälter gelangen. Auf diese Art und Weise können die Luftkeime wirksam zurückgehalten werden. Ferner kann die Luft auf ihrem Eintrittsweg an absorbierenden oder adsorbierenden Materialien vorbeigeführt werden, die die Luftkeime festhalten. Hierbei können insbesondere elektrostatische Kräfte ausgenutzt werden. Weiterhin kann auch die Luftentkeimung durch oligodynamisch wirksame Substanzen, die in dem Eintrittsweg der Luft angeordnet sind, erreicht werden. Wichtig ist auch, daß diese Mittel kombiniert werden können, z.B. werden Keime absorbiert an Mittel, die gleichzeitig mikrobizide Eigenschaften aufweisen.

Ein weiterer Vorteil, der sich aus der getrennten Entkeimung des Fluids und der Luft ergibt, ist, daß Substanzen, die zur Luftentkeimung verwendet werden, mit dem Fluid nicht in Berührung kommen. Dadurch wird verhindert, daß irgendwelche produktfremden Stoffe in das Fluid gelangen und damit dem Verbraucher zugeführt werden.

Die Abgabevorrichtung kann eine Pumpe sein, die auf einen herkömmlichen Vorratsbehälter aufgesetzt ist. Dabei können die zumindest in einem Teil getrennt geführten Fluidausgangs- und Lufteingangswege in der Pumpe integriert sein.

Ferner kann für die Abgabe des Fluids der Vorratsbehälter als eine Quetschflasche ausgebildet sein, wobei in diesem Fall vorteilhafterweise am Fluidausgang ein Ventil vorgesehen ist.

Im Folgenden sollen Ausführungsbeispiele der vorliegenden Erfindung anhand der angefügten Zeichnungen detailliert erläutert werden.

Fig. 1 zeigt als Beispiel eine Abgabevorrichtung, bei der Mittel zur Entkeimung des Fluids und getrennt davon Mittel zur Entkeimung der einströmenden Luft verwendet werden.

Fig. 2 zeigt ein weiteres Ausführungsbeispiel, bei dem als Abgabevorrichtung der Vorratsbehälter als Quetschflasche ausgebildet ist.

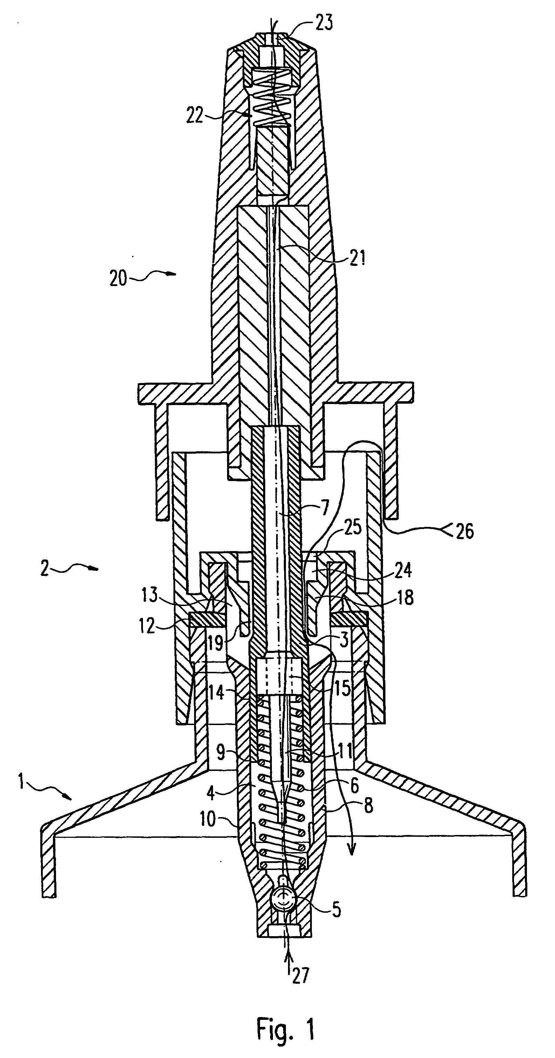

In einem ersten in Fig. 1 dargestellten Ausführungsbeispiel wird als Abgabevorrichtung eine Saug/Druckpumpe 2 verwendet, deren Aufbau anhand der Zeichnung erläutert wird. Die Saug/Druckpumpe 2 ist mittels des Dichtringes 12 dichtend auf dem hier nur teilweise dargestellten Fluidvorratsbehälter 1 aufgesetzt. Im Druckzylinder 8 läuft ein Kolben 3 mit einem axialen Pumpkanal 7. Der Kolben 3 wird von einer Feder 6 an einem Anschlag auf seiner oberen Ruheposition gehalten. Zwischen dem Kolben 3 und dem Kugelventil 5 befindet sich die Druckkammer 4, die mit dem axialen Pumpkanal 7 verbunden ist. Der Kolben 3 hat einen kleineren Außendurchmesser als der Innendurchmesser des Druckzylinders 8, so daß ein Spalt 14 zwischen Kolbenaußenwand und Zylinderinnenwand verbleibt, der nach unten jedoch von der umfangseitigen Dichtlippe 9 des Kolbens verschlossen wird. Im unteren Bereich der Druckkammer 4 weist der Druckzylinder 8 einen Abschnitt 10 mit größerem Innendurchmesser auf, in dem die Dichtlippe 9 nicht dichtend wirkt. Ein auf dem Kolben 3 aufgesetztes Betätigungselement 20 weist ein Steigrohr 21 mit einem Überdruckventil 22 zur Abgabe des Fluids durch eine Auslaßöffnung 23 auf. In der in der Zeichnung dargestellten oberen Ruheposition des Kolbens 3 dichtet die Dichtlippe 9 die Druckkammer 4 gegenüber den Öffnungen 13 zum Fluidbehälter 1 ab. Der Stößel 11 ist fest mit dem Kolben 3 verbunden, wobei der Abschnitt 15 mit sternförmigem Querschnitt eine Verbindung zwischen Druckkammer 4 und Pumpkanal 7 freiläßt. In der Ruheposition der Pumpe hat der Stößel 11 einen Abstand vom Kugelventil 5, so daß dieses sich bei Überdruck in der Druckkammer 4 gegenüber dem Fluidbehälter 1 öffnet und bei Unterdruck schließt. Der Weg des Fluids aus dem Fluidbehälter 1 durch die Abgabevorrichtung 2 ist durch den bei 27 beginnenden Pfeil schematisch dargestellt. Bei geöffnetem Kugelventil 5 tritt das Fluid durch dieses in den Pumpkanal 7 ein und gelangt durch das geöffnete Überdruckventil 22 zur Auslaßöffnung 23. Um eine Kontamination des Vorratsfluids in dem Fluidbehälter 1 zu verhindern, sind auf dem eben beschriebenen Austrittsweg des Fluids oligodynamisch wirksame Substanzen angeordnet. So können diese Substanzen beispielsweise auf der Feder 6, den Innenwandungen des Pumpkanals 7, im Überdruckventil 22 oder an der Austrittsöffnung 23 angeordnet sein.

Zum Druckausgleich im Fluidbehälter 1 bei der Abgabe des Fluids tritt Luft von der Umgebung bei 26 in die Abgabevorrichtung ein, gelangt dann, wie in der Zeichnung anhand des Pfeils schematisch dargestellt, in den Fluidbehälter 1. Hierzu befindet sich zumindest auf einem Teil des Umfangs zwischen dem Kolben 3 und dem Abschnitt 18 ein Spalt 19. Zur Entkeimung der eintretenden Luft sind im Bereich des Lufteintrittsweges Mittel vorgesehen, die die Luft entkeimen. Als solche Mittel kommen ein Sterilfilter, eine Membran, durch die die Luft durch Permeation gelangt, keimabsorbierende oder keimadsorbierende Materialien, oligodynamisch wirksame oder mikrobizide Substanzen sowie Kombinationen davon in Frage. Beispielsweise kann im Bereich 24 ein Sterilfilter 25, durch den die Luft auf ihrem Eintrittsweg gelangen muß, angeordnet sein. Ferner eignet sich der sehr eng ausgebildete Spalt 19 dazu, z.B. metallisches Silber als oligodynamisch wirksame Substanz anzubringen.

Es soll hervorgehoben werden, daß die Entkeimung des Fluids getrennt von der Entkeimung der Luft vorgenommen wird. Dadurch ist es möglich, verschiedene Mittel zur Fluid- bzw. Luftentkeimung bereitzustellen und selektiv einzusetzen. Weiterhin sind die Abgabevorrichtungen dieser Ausführungsbeispiele so ausgeführt, daß sie an beliebige herkömmliche Vorratsflaschen angepaßt werden können und verschiedene Pumpversionen für vertikale und horizontale Ausbringung mit diesem System umgerüstet werden können.

Das Fluid im Vorratsbehälter wird auf preiswerte Art vor Kontamination wirksam geschützt, so daß auf Konservierungsmittel verzichtet werden kann. Konservierungsmittel sind bedenliche Substanzen und führen zu unerwünschten Nebenwirkungen.

## Patentansprüche

1. Abgabevorrichtung für Fluide aus einem Vorratsbehälter (1), bei der das Fluid durch einen Fluidausgang (23) mit Hilfe einer einen Kolben aufweisenden Pumpe (2) abgegeben wird und bei der während der Abgabe des Fluids Luft zum Druckausgleich in den Vorratsbehälter (1) durch einen Lufteingang einströmt, der die Pumpe (2) umgibt und in dem ein Sterilfilter (25) zur Sterilisation, Entkeimung oder Keimreduktion der Luft vorgesehen ist, wobei der Lufteingang durch einen eng ausgebildeten Spalt (19) zwischen dem Umfang des Kolbens (3) der Pumpe (2) und einem Gehäuseabschnitt (18) gebildet ist,
**dadurch gekennzeichnet,**
**daß** zur Sterilisation, Entkeimung oder Keimreduktion des Fluids im Fluidausgangsbereich Oberflächen oder Einrichtungen, die mit dem Fluid in Berührung kommen mit Substanzen versehen sind, die eine oligodynamische Wirkung oder keimreduzierende Eigenschaften aufweisen,
und **daß** in dem Spalt (19) metallisches Silber als oligodynamisch wirksame Substanz angebracht ist.

2. Abgabevorrichtung für Fluide aus einem Vorratsbehälter (1), bei der das Fluid durch einen Fluidausgang (23) mit Hilfe einer einen Kolben aufweisenden Pumpe (2) abgegeben wird und bei der während der Abgabe des Fluids Luft zum Druckausgleich in den Vorratsbehälter (1) durch einen Lufteingang einströmt, der die Pumpe (2) umgibt und in dem ein Sterilfilter (25) zur Sterilisation, Entkeimung oder Keimreduktion der Luft vorgesehen ist, wobei der Lufteingang durch einen eng ausgebildeten Spalt (19) zwischen dem Umfang des Kolbens (3) der Pumpe (2) und einem Gehäuseabschnitt (18) gebildet ist,
**dadurch gekennzeichnet,**
**daß** zur Sterilisation, Entkeimung oder Keimreduktion des Fluids im Fluidausgangsbereich Oberflächen oder Einrichtungen, die mit dem Fluid in Berührung kommen mit Substanzen versehen sind, die eine oligodynamische Wirkung oder keimreduzierende Eigenschaften aufweisen,
und **daß** der Sterilfilter (25) im Bereich (24) vor dem Spalt (19) zwischen dem Umfang des Kolbens (3) und dem Gehäuseabschnitt (18) angeordnet ist.

3. Abgabevorrichtung nach Anspruch 1 oder 2,
**dadurch gekennzeichnet,**
**daß** die oligodynamisch wirksame Substanz ein Schwermetall und/oder eine Schwermetall-Legierung ist die in metallischer und/oder ionischer Form wirksam ist.

4. Abgabevorrichtung nach einem der Ansprüche 1 oder 2,
**dadurch gekennzeichnet,**
**daß** die oligodynamisch wirksame Substanz Silber ist.

5. Abgabevorrichtung nach einem der vorhergehenden Ansprüche,
**dadurch gekennzeichnet,**
**daß** die Mittel zur Sterilisation, Entkeimung oder Keimreduktion der Luft bzw. des Fluids unterschiedlich sind.

6. Abgabevorrichtung nach einem der Ansprüche 2 bis 5,
**dadurch gekennzeichnet,**
**daß** in dem sehr eng ausgebildeten Spalt (19) metallisches Silber als oligodynamisch wirksame Substanz angebracht ist.

7. Abgabevorrichtung nach einem der vorhergehenden Ansprüche,
**dadurch gekennzeichnet,**
**daß** die Mittel zur Entkeimung der eintretenden Luft in dem Sterilfilter (25) oligodynamisch wirksame Substanzen sind.

## Claims

1. Dispenser for fluids from a storage container (1), in which the fluid is dispensed through a fluid outlet (23) with the aid of a pump (2) having a piston and in which, while the fluid is being dispensed, in order to equalise the pressure air flows into the storage container (1) through an air inlet which surrounds the pump (2) and in which a sterile filter (25) is provided for sterilisation of, degermination of or reduction of the germs in the air, the air inlet being formed by a narrowly constructed gap (19) between the periphery of the piston (3) of the pump (2) and a casing section (18),
**characterised in that,**
for sterilisation of, degermination of or reduction of the germs in the fluid in the fluid outlet region, surfaces or devices which come into contact with the fluid are provided with substances which have an oligodynamic effect or germ-reducing properties, and **in that** metallic silver is provided in the gap (19) as the oligodynamically effective substance.

2. Dispenser for fluids from a storage container (1), in which the fluid is dispensed through a fluid outlet (23) with the aid of a pump (2) having a piston and in which, while the fluid is being dispensed, in order to equalise the pressure air flows into the storage container (1) through an air inlet which surrounds the pump (2) and in which a sterile filter (25) is provided for sterilisation of, degermination of or reduction of the germs in the air, the air inlet being formed by a narrowly constructed gap (19) between the periphery of the piston (3) of the pump (2) and a casing section (18),
**characterised in that,**
for sterilisation of, degermination of or reduction of the germs in the fluid in the fluid outlet region, surfaces or devices which come into contact with the fluid are provided with substances which have an oligodynamic effect or germ-reducing properties,
and **in that** the sterile filter (25) is arranged in the region (24) in front of the gap (19) between the periphery of the piston (3) and the casing section (18).

3. Dispenser according to Claim 1 or 2,
**characterised in that**
the oligodynamically effective substance is a heavy metal and/or a heavy-metal alloy which is effective in metallic and/or ionic form.

4. Dispenser according to one of Claims 1 and 2,
**characterised in that**
the oligodynamically effective substance is silver.

5. Dispenser according to one of the preceding claims,
**characterised in that**
the means for sterilisation of, degermination of or reduction of the germs in the air and the fluid are different.

6. Dispenser according to one of Claims 2 to 5,
**characterised in that**
metallic silver is provided in the very narrowly constructed gap (19) as the oligodynamically effective substance.

7. Dispenser according to one of the preceding claims,
**characterised in that**
the means for degerminating the incoming air in the sterile filter (25) are oligodynamically effective substances.

## Revendications

1. Dispositif distributeur de fluides à partir d'un réceptacle de réserve (1), dans lequel le fluide est délivré par l'intermédiaire d'une sortie (23) de fluide, à l'aide d'une pompe (2) présentant un piston, et dans lequel, au cours de la délivrance du fluide, de l'air afflue dans le réceptacle de réserve (1) en vue de l'équilibrage de pression, en empruntant une admission d'air qui entoure la pompe (2) et dans laquelle un filtre stérile (25) est prévu pour la stérilisation, la suppression des germes ou la réduction des germes dans l'air, l'admission d'air étant formée par un interstice (19) de réalisation étroite entre le pourtour du piston (3) de la pompe (2) et une zone de boîtier (18),
**caractérisé par le fait**
**que**, pour assurer la stérilisation, la suppression des germes ou la réduction des germes dans le fluide, des surfaces ou dispositifs entrant en contact avec le fluide sont muni(e)s, dans la région de la sortie dudit fluide, de substances à effet oligodynamique ou à propriétés réductrices de germes ;
et par le fait que de l'argent à l'état métallique est déversé dans l'interstice (19), en tant que substance oligodynamiquement opérante.

2. Dispositif distributeur de fluides à partir d'un réceptacle de réserve (1), dans lequel le fluide est délivré par l'intermédiaire d'une sortie (23) de fluide, à l'aide d'une pompe (2) présentant un piston, et dans lequel, au cours de la délivrance du fluide, de l'air afflue dans le réceptacle de réserve (1) en vue de l'équilibrage de pression, en empruntant une admission d'air qui entoure la pompe (2) et dans laquelle un filtre stérile (25) est prévu pour la stérilisation, la suppression des germes ou la réduction des germes dans l'air, l'admission d'air étant formée par un interstice (19) de réalisation étroite entre le pourtour du piston (3) de la pompe (2) et une zone de boîtier (18),
**caractérisé par le fait**
**que**, pour assurer la stérilisation, la suppression des germes ou la réduction des germes dans le fluide, des surfaces ou dispositifs entrant en contact avec le fluide sont muni(e)s, dans la région de la sortie dudit fluide, de substances à effet oligodynamique ou à propriétés réductrices de germes ;
et par le fait que le filtre stérile (25) est placé dans la région (24) précédant l'interstice (19), entre le pourtour du piston (3) et la zone de boîtier (18).

3. Dispositif distributeur selon la revendication 1 ou 2,
**caractérisé par le fait**
**que** la substance oligodynamiquement opérante est un métal lourd et/ou un alliage de métaux lourds, agissant sous forme métallique et/ou ionique.

4. Dispositif distributeur selon l'une des revendications 1 ou 2,
**caractérisé par le fait**
**que** la substance oligodynamiquement opérante est de l'argent.

5. Dispositif distributeur selon l'une des revendications précédentes,
**caractérisé par le fait**
**que** les moyens, respectivement prévus pour la stérilisation, la suppression des germes ou la réduction des germes dans l'air ou dans le fluide, sont de types différents.

6. Dispositif distributeur selon l'une des revendications 2 à 5,
**caractérisé par le fait**
**que** de l'argent à l'état métallique est déversé, dans l'interstice (19) de réalisation très étroite, en tant que substance oligodynamiquement opérante.

7. Dispositif distributeur selon l'une des revendications précédentes,
**caractérisé par le fait**
**que** les moyens de suppression des germes de l'air affluant, dans le filtre stérile (25), sont des substances oligodynamiquement opérantes.
